# EUROPEAN PATENT APPLICATION

(11) **EP 3 815 614 A1**
(43) Date of publication of application: **05.05.2021**
(21) Application number: 19205548.1
(22) Date of filing: 28.10.2019
(51) Int. Cl.: A61B 8/08, A61B 8/12, A61B 8/00, G01S 15/89, A61B 34/20, A61B 90/00, A61B 8/06

(54) **ULTRASOUND DEVICE TRACKING**

(71) Applicant: Koninklijke Philips N.V., 5656 AG Eindhoven (NL)
(72) Inventor: VAN DER HORST, Arjen, 5656 AE Eindhoven (NL); STAPERT, Hendrik Roelof, 5656 AE Eindhoven (NL)
(74) Representative: Philips Intellectual Property & Standards

(57) **Abstract**

The invention provides an ultrasound system comprising an ultrasound probe, adapted to transmit and receive ultrasound signals, and an interventional device for insertion into a vessel of a subject. The interventional device includes an ultrasound transducer adapted to acquire a first set of ultrasound data at a first ultrasound frequency, wherein the first set of ultrasound data relates to flow data. The ultrasound system is further adapted acquire a second set of ultrasound data by way of the ultrasound probe or the interventional device at a second ultrasound frequency different from the first ultrasound frequency, wherein the second ultrasound data relates to interventional device positioning data.

## Description

### FIELD OF THE INVENTION

The invention relates to the field of ultrasound imaging, and more specifically the field of ultrasound device tracking.

### BACKGROUND OF THE INVENTION

Percutaneous valve interventions are increasingly becoming the treatment of choice over open-heart valve surgery. In particular, the number of catheter-based mitral and tricuspid valve repairs, which are technically more challenging than aortic valve replacement, are increasing rapidly.

One of the challenges during cardiac valve repair is deciding at what point the repair is sufficiently successful to stop the procedure. The most common methods used for this are: transesophageal echocardiography (TEE); transthoracic echocardiography (TTE); and intracardiac echocardiography (ICE). However, it is often difficult to obtain an accurate quantification of the blood flow through the valve for assessing the success of the procedure using these methods. Other medical procedures in the vasculature, including both cardiac and peripheral vasculature procedures, may also benefit from the ability to assess the blood flow in the vasculature.

There is therefore a need for an improved means of assessing the blood flow in the vasculature.

### SUMMARY OF THE INVENTION

The invention is defined by the claims.

According to examples in accordance with an aspect of the invention, there is provided an ultrasound system, the system comprising:
an ultrasound probe adapted to transmit and receive ultrasound signals; and
an interventional device for insertion into a vessel of a subject 100 comprising:
   an ultrasound transducer, wherein the ultrasound transducer is adapted to:
   acquire a first set of ultrasound data at a first ultrasound frequency, wherein the first set of ultrasound data relates to flow data,

wherein the ultrasound system is further adapted to acquire a second set of ultrasound data by way of the ultrasound probe or the interventional device at a second ultrasound frequency different from the first ultrasound frequency, wherein the second ultrasound data relates to interventional device positioning data.

The system provides for a means of performing flow measurements whilst also tracking the position of the interventional device within a vessel. Thus, the functionality of a flow measurement and device tracking system may be increased without increasing its complexity.

In an embodiment, the first ultrasound frequency and the second ultrasound frequency are resonant frequencies of the ultrasound transducer, and wherein the second ultrasound frequency is an operating frequency of the ultrasound probe.

By operating the ultrasound transducer at resonant frequencies, the amplitude of the displacement of the ultrasound transducer is increased for less force, which increases the effectiveness of the ultrasound imaging catheter when in liquid, such as when located in the vessel of a subject.

In an embodiment, the first ultrasound frequency is greater than the second ultrasound frequency.

In an embodiment, the ultrasound transducer is a CMUT.

In this way, the operating frequency of the ultrasound transducer may be tuned by altering the bias voltage applied to the CMUT.

In an embodiment, the ultrasound imaging catheter comprises a plurality of ultrasound transducers.

In a further embodiment, each ultrasound transducer of the plurality of ultrasound transducers is controlled individually.

In this way, the ultrasound transducers may be controlled to perform electronic steering of the ultrasound beam.

In a yet further embodiment, the plurality of ultrasound transducers is arranged into a first subgroup and a second subgroup, wherein the first subgroup is adapted to only transmit ultrasound waves and the second subgroup is adapted to only receive ultrasound waves.

In this way, the accuracy of the interventional device positioning data may be increased.

In an embodiment, the plurality of ultrasound transducers is adapted to acquire a third set of ultrasound data comprising B-mode data.

In this way, an additional set of data may be used to monitor the position of the ultrasound imaging catheter within a volume.

In an embodiment, the ultrasound system further comprises a processor wherein the processor is adapted to generate a flow map based on a combination of the first set of ultrasound data, relating to flow data, and the second set of ultrasound data, relating to interventional device positioning data.

In an embodiment, the ultrasound probe is adapted to acquire an ultrasound image, wherein the ultrasound image comprises a view of the vessel of the subject, and wherein the processor is further adapted to generate a visual representation of the flow map and overlay the visual representation of the flow map on the ultrasound image.

In an embodiment, the system further comprises:
a feedback mechanism adapted to generate an interventional device steering instruction based on the first set of ultrasound data and/or the second set of ultrasound data; and
a steering mechanism adapted to steer the interventional device based on the steering instruction.

In this way, the ultrasound imaging catheter may be steered to the optimal imaging positon.

According to examples in accordance with an aspect of the invention, there is provided a method for performing ultrasound imaging using an ultrasound system comprising an interventional device, having an ultrasound transducer, and an ultrasound probe, the method comprising:
acquiring a first set of ultrasound data at a first ultrasound frequency by way of the interventional device, wherein the first set of ultrasound data relates to flow data; and
acquiring a second set of ultrasound data at a second ultrasound frequency different from the first ultrasound frequency by way of the interventional device or the ultrasound probe, wherein the second ultrasound data relates to catheter positioning data.

In an embodiment, the method further comprises steering the interventional device based on the first set of ultrasound data and/or the second set of ultrasound data. In an embodiment, the method further comprises:
combining the first set of ultrasound data with the second set of ultrasound data; and
generating a flow map of the vessel based on the combined first set of ultrasound data and second set of ultrasound data.

According to examples in accordance with an aspect of the invention, there is provided a computer program comprising computer program code means which is adapted, when said computer program is run on a computer, to implement the methods as described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an ultrasound diagnostic imaging system to explain the general operation;
Fig. 2 shows example of an ultrasound system according to an aspect of the invention;
Fig. 3 shows an example of an interventional device;
Fig. 4 shows an example of the interventional device of Fig. 3 disposed within a vessel of a subject;
Fig. 5 shows several implementation options for the ultrasound transducer of the interventional device;
Fig. 6 shows an example of an interventional device with a plurality of ultrasound transducer elements;
Fig. 7 shows a further implementation of the interventional device; and
Fig. 8 shows a method for performing ultrasound imaging using an ultrasound imaging catheter.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an ultrasound system comprising an ultrasound probe, adapted to transmit and receive ultrasound signals, and an interventional device for insertion into a vessel of a subject. The interventional device includes an ultrasound transducer adapted to acquire a first set of ultrasound data at a first ultrasound frequency, wherein the first set of ultrasound data relates to flow data. The ultrasound system is further adapted acquire a second set of ultrasound data by way of the ultrasound probe or the interventional device at a second ultrasound frequency different from the first ultrasound frequency, wherein the second ultrasound data relates to interventional device positioning data.

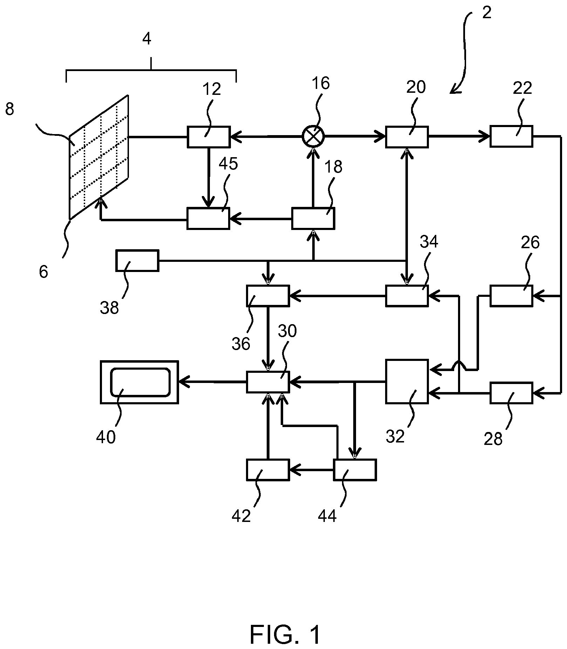

The general operation of an exemplary ultrasound system will first be described by means of a non-limiting example with reference to Fig. 1.

The system comprises an array transducer probe 4 which has a transducer array 6 for transmitting ultrasound waves and receiving echo information. The transducer array 6 may comprise CMUT transducers; piezoelectric transducers, which may be formed of piezoelectric materials such as PZT or PVDF; or any other suitable ultrasound transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

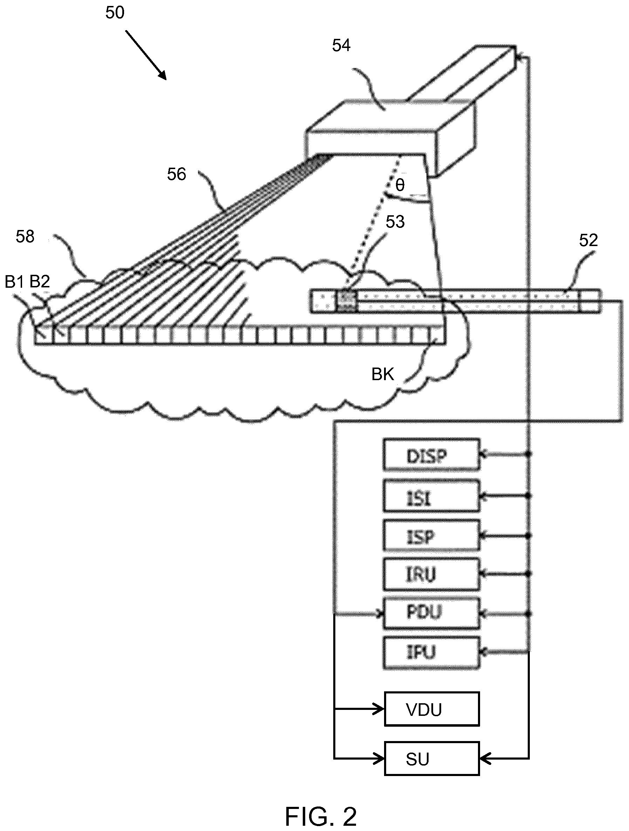

Fig. 2 shows an example of an ultrasound system according to an aspect of the invention, which may employ any of the aspects described above with reference to Fig. 1.

In particular, Fig. 2 shows an ultrasound-based position determination system 50 in which the interventional device for insertion into the vessel of a subject described herein may be used. The interventional device 52 may be any device suitable for insetion into a vessel of a subject having an ultrasound transducer 53. For example, the intervention device may be: a catheter; a guidewire; a needle; and the like.

The ultrasound system 50 includes an interventional device 52; an ultrasound probe 54 configured to generate an ultrasound field 56, an image reconstruction unit IRU configured to provide a reconstructed ultrasound image corresponding to the ultrasound field 56 of the beamforming ultrasound probe 54; a position determination unit PDU configured to compute a position of the ultrasound transducer 53 of the interventional device 52 with respect to the ultrasound field 56 based on ultrasound signals transmitted between the ultrasound probe 54 and the ultrasound transducer 53.

The ultrasound system may also include an icon providing unit IPU configured to provide an icon in a reconstructed ultrasound image based on the computed position of the ultrasound transducer 53.

In Fig. 2, the ultrasound probe 54 is in communication with an image reconstruction unit IRU, an imaging system processor ISP, an imaging system interface ISI and a display DISP. Together, the ultrasound probe 54, IRU, ISP, ISI and DISP may be provided as part of a conventional ultrasound imaging system, such as the system described above with reference to Fig. 1. The units IRU, ISP, ISI and DISP are conventionally located in a console that is in wired or wireless communication with the ultrasound probe. Alternatively, some of units IRU, ISP, ISI and DISP may be incorporated within the ultrasound probe.

Fig. 2 illustrates an ultrasound probe 54 that includes a linear ultrasound transducer array that transmits and receives ultrasound signals within an ultrasound field 56, which intercepts a region of interest 58, such as the vessel of a subject that is under investigation.

In the example shown in Fig. 2, the ultrasound field is fan shaped and includes multiple ultrasound beams [B1, B2, ..., BK] that together provide the illustrated image plane. It should be noted that whilst a fan-shaped beam is illustrated for the purposes of illustration, any suitable shape of ultrasound field may be used.

In use the above described ultrasound imaging system may be operated in the following way. An operator may plan an ultrasound procedure via imaging system interface ISI. Once an operating procedure is selected, imaging system interface ISI triggers imaging system processor ISP to execute application-specific programs that generate and interpret the signals transmitted to and detected by the ultrasound probe 54. A memory (not shown) may be used to store such programs. The memory may, for example, store ultrasound beam control software that is configured to control the sequence of ultrasound signals transmitted by and/or received by the ultrasound probe.

Image reconstruction unit IRU, which may alternatively form part of imaging system processor ISP, provides a reconstructed ultrasound image corresponding to the ultrasound field 56 of the ultrasound probe. The image is subsequently displayed on display DISP. The reconstructed image may for example, be an ultrasound B-mode image, a C-mode image or a Doppler mode image, or indeed any ultrasound image.

Also shown in Fig. 2 is interventional device 52 that includes an ultrasound transducer 53. The interventional device may be tracked with respect to the ultrasound field 56 based on signals provided by position determination unit PDU and icon providing unit IPU. These units are in communication with one another and units BUIP, IRU, ISP, ISI and DISP. One or more of units PDU and IPU may be incorporated within a memory or a processor of the ultrasound system.

The interventional device 52 is further connected to velocity determination unit VDU, which is adapted to determine a velocity of the blood flow within a vessel in which the interventional device may be located based on ultrasound signals transmitted and received by the ultrasound transducer 53. The ultrasound signals used for the velocity determination are generated at a first frequency and the ultrasound signals used for the position determination are generated at a second frequency, different from the first frequency in order to reduce interference.

The ultrasound system 50 may also include a synchronization unit SU, which is adapted to synchronize the data from the PDU and the VDU, in order to match velocity data with position data along the length of a vessel. These units are in communication with one another and units BUIP, IRU, ISP, ISI and DISP. One or more of units VDU and SU may be incorporated within a memory or a processor of the ultrasound system.

In use, the position of the ultrasound transducer 53 of the interventional device 52 is computed with respect to ultrasound field 56 by position determination unit PDU based on ultrasound signals transmitted between ultrasound probe 54 and the ultrasound transducer.

In one example, the ultrasound transducer 53 is a detector that receives ultrasound signals corresponding to the ultrasound beams. In this case, position determination unit PDU identifies the position of the ultrasound transducer by correlating the ultrasound signals emitted by the ultrasound probe with the ultrasound signals detected by the ultrasound transducer. Icon providing unit IPU may subsequently provide an icon in the reconstructed ultrasound image based on the computed position of the ultrasound transducer.

More specifically, the best fit position of the ultrasound transducer with respect to the ultrasound field may be determined based on: the amplitudes of the ultrasound signals corresponding to each ultrasound beam that are detected by the ultrasound transducer; and the time delay, i.e. time of flight, between emission of each ultrasound beam and its detection by the ultrasound transducer.

For example, when the ultrasound transducer 53 is in the vicinity of the ultrasound field 56, ultrasound signals from the nearest of ultrasound beam to the ultrasound transducer will be detected with a relatively larger amplitude compared to more distant beams, which will be detected with relatively smaller amplitudes. Typically the beam that is detected with the largest amplitude is identified as the one that is closest to the ultrasound transducer. This beam defines the in-plane angle θ between the ultrasound probe 54 and the ultrasound transducer 53.

The corresponding range depends upon the time delay, i.e. the time of flight, between the emission of the largest-amplitude ultrasound beam and its subsequent detection. The range is determined by multiplying the time delay by the speed of ultrasound propagation. Thus, the range and corresponding in-plane angle, θ, of the beam detected with the largest amplitude can be used to identify the best-fit position of the ultrasound transducer with respect to the ultrasound field.

In an alternative configuration, the ultrasound transducer 53 may be an emitter that emits one or more ultrasound pulses. Such pulses may, for example, be emitted during tracking frames that are interleaved between the imaging frames of the 1 ultrasound imaging system. In such a tracking frame, the ultrasound probe 54 may operate in a receive-only mode, in which it listens for ultrasound signals originating from the vicinity of the ultrasound field 56.

In other words, the ultrasound probe is configured as a one-way receive-only beamformer during such tracking frames. Position determination unit PDU identifies from which ultrasound beam [B1,..., BK], the pulse(s) would have originated from by applying delays to the receiver elements of the ultrasound probe. As in the detector configuration above, position determination unit PDU may use a correlation procedure that, based on the maximum amplitude and time of flight, identifies the closest beam to the position at which the ultrasound signal was emitted, and the corresponding range to the ultrasound transducer 53. Icon providing unit IPU subsequently provides an icon in the reconstructed ultrasound image based on the identified position of the ultrasound transducer. Thus, when the ultrasound transducer is an ultrasound emitter, a correlation procedure may again be used to determine the best-fit position of piezoelectric transducer 53 respective ultrasound field UF for each tracking frame.

In another configuration, the ultrasound transducer 53 may be configured to act as both a receiver and an emitter, or include a separate receiver and emitter. In such a configuration, the ultrasound transducer may be triggered to emit one or more ultrasound pulses upon receipt of an ultrasound signal from the ultrasound probe. In this way the pulse(s) emitted by the ultrasound transducer during an imaging mode are received by the ultrasound probe appear as an echo in the reconstructed ultrasound at an in-plane angular position, i.e. in an image line, that corresponds to the relevant ultrasound beam.

The ultrasound transducer 53, thus appears as a bright spot in the reconstructed image. Position determination unit PDU may subsequently identify this bright spot in the reconstructed image and compute a position of the ultrasound transducer with respect to the ultrasound field.

The ultrasound probe described above may be any type of probe capable of capturing ultrasound data. For example, the ultrasound probe may be a standard handheld imaging probe or an ultrasound imaging catheter, a catheter being a thin tube adapted to be inserted into the body of a subject, for example within a blood vessel or heart..

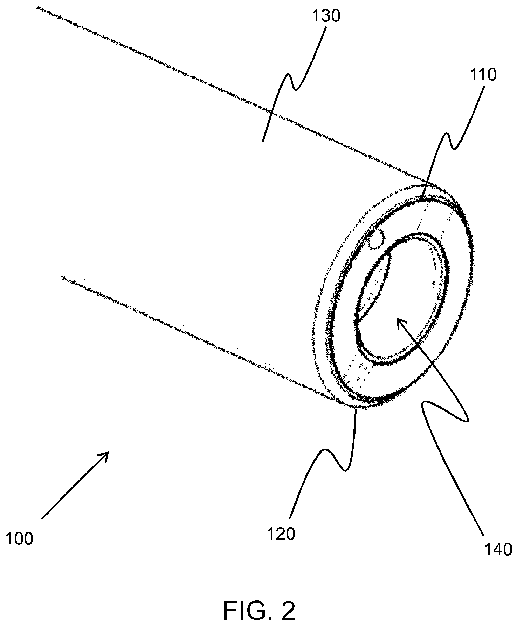

Fig. 3 shows an interventional device 100 according to an aspect of the invention.

In the example shown in Fig. 3, an ultrasound transducer 110 is provided at the device tip 120 located at an end of a device wall 130, which defines a lumen 140.

The ultrasound transducer 110 is adapted to acquire a first set of ultrasound data at a first ultrasound frequency. In other words, the ultrasound transducer is actuated at a first frequency in order to generate ultrasound signals at said first frequency.

When the ultrasound imaging catheter is provided within a blood vessel of a subject, the first set of ultrasound data relates to flow data. The flow data may comprise any data relating to blood flow, such as: blood velocity; turbulence; blood pressure; heart rate; vessel diameter; and the like.

In addition, the ultrasound transducer 110 may be adapted to acquire a second set of ultrasound data at a second ultrasound frequency different from the first ultrasound frequency in the manner described above with respect to Fig. 2. As described above, the ultrasound transducer is actuated at a second frequency in order to generate ultrasound signals at said second frequency. The second frequency may be any frequency different from the first frequency.

When the interventional device is provided within a blood vessel of a subject, the second ultrasound data may relate to interventional device positioning data. The interventional device data may be any data relating to the location of the interventional device.

In other words, there is provided an interventional device having an ultrasound transducer that is adapted to operate at two different frequencies for two different purposes. Put another way, the interventional device comprises a single component type, being the ultrasound transducer, which is operated such that it may perform multiple functions without increasing the hardware requirements of the ultrasound imaging catheter.

The interventional device is adapted to capture both flow data, relating to a blood flow of the subject, and interventional device positioning data, which may be used to track the position of the interventional device in an ultrasound volume.

As described above with respect to Fig. 2, the interventional device positioning data is acquired by a combination of the ultrasound transducer of the interventional device and a separate ultrasound probe.

A combination of catheter position tracking and flow sensing may be performed by exploiting multiple resonance peaks of the ultrasound transducer. For example, a given ultrasound transducer may have a first resonance peak of 12MHz, which may be used for flow velocity sensing and a second resonance peak at 6MHz, which may be used for capturing position data of the ultrasound imaging catheter. For example, the first resonance peak may be in a range of 11MHz to 13MHz and the second resonance peak may be in a range of 5MHz to 7MHz.

By operating the ultrasound transducer at a resonance peak, the interventional device may more efficiently generate ultrasound waves within a vessel of a subject, and in particular when the ultrasound transducer is submerged in a liquid. By operating the transducer at a resonance peak, it is possible to generate substantial vibrations, and so ultrasonic waves, at a lower energy input, thereby increasing the efficiency of the ultrasound imaging catheter.

A higher frequency resonance peak, such as 12MHz, may be selected for obtaining the first set of ultrasound data, relating to flow data, in order to maximize the backscatter from the blood cells and a lower resonance peak, such as 6MHz may be selected for obtaining the second set of ultrasound data, relating to catheter position data.

The second set of ultrasound data may be obtained at a frequency of 6MHz, which may be within the frequency range of typical imaging devices used for procedures such as: transesophageal echocardiography (TEE); transthoracic echocardiography (TTE); and intracardiac echocardiography (ICE).

Put another way, the ultrasound transducer may be controlled to operate at a response frequency of a secondary imaging device, which may then be used in the tracking of the position of the interventional device within the subject.

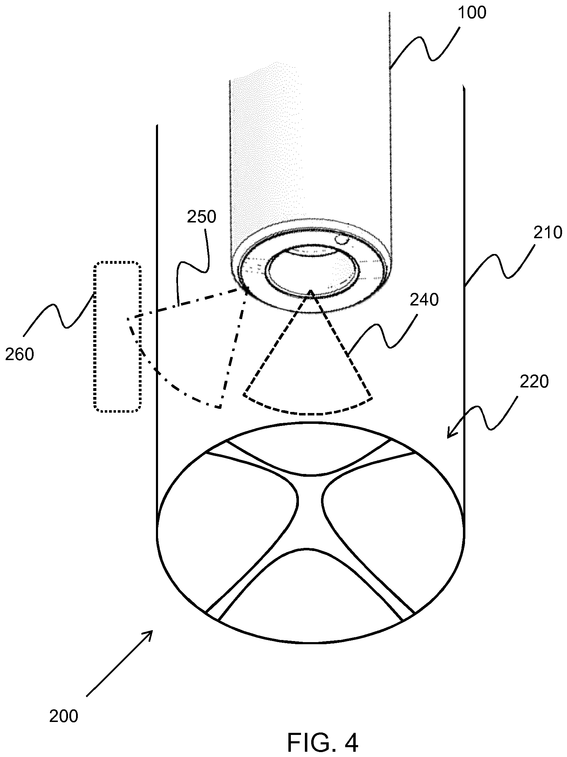

Fig. 4 shows an example 200 of the interventional device 100 of Fig. 2 within a vessel 210 of the subject in the proximity of a valve 220.

In this example, the interventional device may be deployed to perform, or aid in the performance of, a valve repair. For example, the interventional device may gather data, such as the flow data, which may be used in informing the decision of a clinician as to when the valve repair is sufficient. For example, the flow data may be visually presented to the clinician in order to provide a real-time view of the flow of blood in the area of interest. In this example, the catheter positioning data may be used by the clinician to help them guide the catheter to the valve needing repair.

Further, the flow data may be combined with the interventional device positioning data in order to generate a flow map of the vessel, or valve, undergoing investigation, or repair. The flow map may be overlain on an ultrasound image to be presented to the user in order to inform their decision. In addition, the interventional device may include a steering element adapted to steer the interventional device based on the first set of ultrasound data, the second set of ultrasound data or an input from the user, for example to maximize the view of the ultrasound transducer used to determine the flow data.

Fig. 3 shows the interventional device 100 generating a first set of ultrasound waves 240, at a first ultrasound frequency, in order to obtain the flow data. Further, the ultrasound imaging catheter is shown generating a second set of ultrasound waves 250, at a second ultrasound frequency, which is received by a secondary imaging device 260, such as the ultrasound probe described above, which may be used to track the position of the interventional device by monitoring the second ultrasound frequency at the surface of the subject's skin. As described above, the secondary imaging device may generate the second set of ultrasound waves at the second frequency, which are then received at the ultrasound transducer of the interventional device.

In the case of valve repair, the ultrasound imaging catheter may also act as a means of delivering the repair device to the valve, for example by way of the catheter lumen 140.

In the examples shown in Fig. 3 and Fig. 4, the interventional 100 comprises an ultrasound transducer 110 in the form of a ring-shaped piezoelectric transducer (PZT) element embedded into the wall of the tip of device. The PZT ring transducer may be connected via electrical leads to a console, which may hold signal generation, signal acquisition, and signal processing elements required for standard pulsed Doppler velocity measurement. A pulsed Doppler imaging scheme may be used to measure the velocity spectrum of a volume area near a valve opening, i.e. the flow data. Based on this velocity information, feedback may be provided to the clinician about the progression of the given procedure, for example the progress of the valve repair.

Rather than a PZT, the ultrasound transducer 110 may be a CMUT. As the operation of a CMUT is controlled by the bias voltage applied to it, it is possible to tune the operating frequency of the ultrasound transducer by altering the bias voltage.

Further, the ultrasound transducer may be located at various different points on the interventional device according to the application of the interventional device. For example, the ultrasound transducer may be provided: at the device wall; within a lumen of the device; at the device tip; or embedded within the device tip.

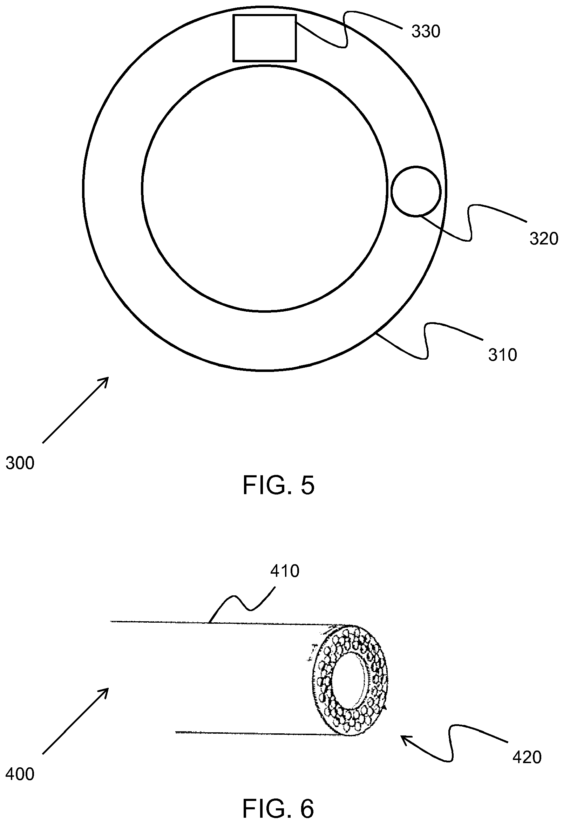

Fig. 5 shows an example 300 of several implementation options for the ultrasound transducer of the interventional device 310.

In the example shown in Fig. 5, the ultrasound transducer may be a round transducer 320 or the ultrasound transducer may be a rectangular transducer 330. Further, a transducer of any other suitable shape may be used as the ultrasound transducer.

Fig. 6 shows an example 400 of an interventional device 410 with a plurality of ultrasound transducer elements 420 located at the device tip.

The plurality of ultrasound transducers 420 may be operated in a number of ways according to the desired operation of the interventional device 410.

For example, each ultrasound transducer of the plurality of ultrasound transducers may be controlled individually. In this way, the plurality of ultrasound transducers may be operated to perform electronic steering of the ultrasound beam. In the case where a secondary imaging device is used to obtain the catheter positioning data, the individual operation of the ultrasound transducer elements provides for a means of determining the orientation of the catheter with respect to the secondary imaging device based on the amplitude and phase differences in the ultrasound signals generated by the plurality of ultrasound transducers.

Alternatively, the plurality of ultrasound transducers may be grouped into a first subgroup and a second subgroup, wherein the first subgroup is adapted to only transmit ultrasound waves and the second subgroup is adapted to only receive ultrasound waves. By operating the ultrasound transducers in sub groups in this way, continuous wave Doppler or hybrid driving schemes are possible in addition to pulsed wave Doppler imaging schemes.

When the interventional device comprises a plurality of ultrasound imaging transducers, the interventional device may be further adapted to acquire a third set of ultrasound data comprising B-mode data. Put another way, a forward looking B-mode image may be made to provide a visualization of, for example, a valve and a means of monitoring the placement of the repair device.

The interventional device may be connected to a feedback mechanism adapted to generate a catheter steering instruction based on the first set of ultrasound data and/or the second set of ultrasound data and a steering mechanism adapted to steer the ultrasound imaging catheter based on the steering instruction.

Put another way, the interventional device may be provided with steering capabilities including a feedback mechanism that steers the catheter towards the most optimal orientation with respect to the blood flow direction. The most optimal orientation may be, for example, where the highest velocities are measured.

In other words, the interventional device may be steerable to enable alignment of the catheter with the flow within the vessel.

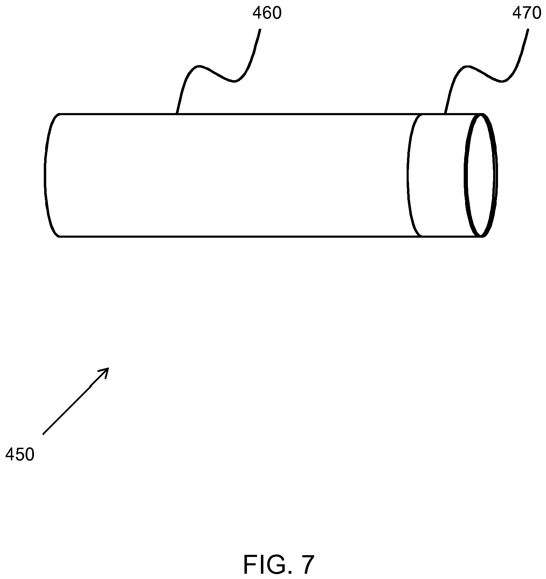

Fig. 7 shows a further implementation 450 of the interventional device 460, wherein the interventional device is a guidewire with an ultrasound transducer 470 located at the tip of the wire.

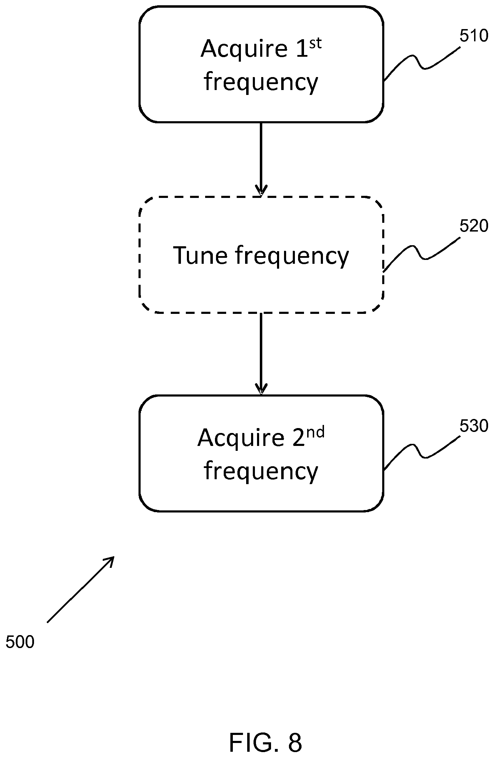

Fig. 8 shows a method 500 for performing ultrasound imaging using an ultrasound imaging catheter.

The method begins in step 510 by acquiring a first set of ultrasound data at a first ultrasound frequency by way of an ultrasound transducer, wherein the first set of ultrasound data relates to flow data.

In step 520, the frequency of the ultrasound transducer may be tuned from the first ultrasound frequency to the second ultrasound frequency. Alternatively, the frequency may be switched in a discrete manner.

In step 530, a second set of ultrasound data is acquired at a second ultrasound frequency different from the first ultrasound frequency by way of the ultrasound transducer, wherein the second ultrasound data relates to catheter positioning data.

In use, the ultrasound transducer of the interventional device may transmit a first set of ultrasound waves at the first frequency in the transmit phase and receive both the echoes of the first set of ultrasound waves and the second set of ultrasound waves at the second frequency simultaneously in the receive phase. In other words, all of the vibrations may be recorded at the ultrasound transducer with a first peak at the first frequency and a second peak at the second frequency.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. An ultrasound system (50), the system comprising:
an ultrasound probe (54) adapted to transmit and receive ultrasound signals; and
an interventional device (52) for insertion into a vessel of a subject comprising:
an ultrasound transducer (53), wherein the ultrasound transducer is adapted to:
acquire a first set of ultrasound data at a first ultrasound frequency, wherein the first set of ultrasound data relates to flow data,
wherein the ultrasound system is further adapted to acquire a second set of ultrasound data by way of the ultrasound probe (54) or the interventional device (52) at a second ultrasound frequency different from the first ultrasound frequency, wherein the second ultrasound data relates to interventional device positioning data.

2. An ultrasound system (50) as claimed in claim 1, wherein the first ultrasound frequency and the second ultrasound frequency are resonant frequencies of the ultrasound transducer, and wherein the second ultrasound frequency is an operating frequency of the ultrasound probe.

3. An ultrasound system (50) as claimed in any of claims 1 to 2, wherein the first ultrasound frequency is greater than the second ultrasound frequency.

4. An ultrasound system (50) as claimed in any of claims 1 to 3, wherein the ultrasound transducer (53) is a CMUT.

5. An ultrasound system (50) as claimed in any of claims 1 to 4, wherein the interventional device comprises a plurality of ultrasound transducers (420).

6. An ultrasound system (50) as claimed in claim 5, wherein each ultrasound transducer of the plurality of ultrasound transducers (420) is controlled individually.

7. An ultrasound system (50) as claimed in any of claims 5 to 6, wherein the plurality of ultrasound transducers (420) is arranged into a first subgroup and a second subgroup, wherein the first subgroup is adapted to only transmit ultrasound waves and the second subgroup is adapted to only receive ultrasound waves.

8. An ultrasound system (50) as claimed in any of claims 5 to 7, wherein the plurality of ultrasound transducers (420) is adapted to acquire a third set of ultrasound data comprising B-mode data.

9. An ultrasound system (50) as claimed in any of claims 1 to 8, wherein the ultrasound system further comprises a processor wherein the processor is adapted to generate a flow map based on a combination of the first set of ultrasound data, relating to flow data, and the second set of ultrasound data, relating to interventional device positioning data.

10. An ultrasound system (50) as claimed in claim 9, wherein the ultrasound probe is adapted to acquire an ultrasound image, wherein the ultrasound image comprises a view of the vessel of the subject, and wherein the processor is further adapted to generate a visual representation of the flow map and overlay the visual representation of the flow map on the ultrasound image.

11. An ultrasound system (50) as claimed in any of claims 1 to 10, wherein the system further comprises:
a feedback mechanism adapted to generate an interventional device steering instruction based on the first set of ultrasound data and/or the second set of ultrasound data; and
a steering mechanism adapted to steer the interventional device based on the steering instruction.

12. A method (500) for performing ultrasound imaging using an ultrasound system comprising an interventional device, having an ultrasound transducer, and an ultrasound probe, the method comprising:
acquiring (510) a first set of ultrasound data at a first ultrasound frequency by way of the interventional device, wherein the first set of ultrasound data relates to flow data; and
acquiring (530) a second set of ultrasound data at a second ultrasound frequency different from the first ultrasound frequency by way of the interventional device or the ultrasound probe, wherein the second ultrasound data relates to catheter positioning data.

13. A method as claimed in claim 12, wherein the method further comprises steering the interventional device based on the first set of ultrasound data and/or the second set of ultrasound data.

14. A method as claimed in any of claims 12 to 13, wherein the method further comprises:
combining the first set of ultrasound data with the second set of ultrasound data; and
generating a flow map of the vessel based on the combined first set of ultrasound data and second set of ultrasound data.

15. A computer program comprising computer program code means which is adapted, when said computer program is run on a computer, to implement the method of any of claims 12 to 14.
